# EUROPÄISCHE PATENTSCHRIFT

(11) **EP 1 722 702 B1**
(45) Veröffentlichungstag und Bekanntmachung des Hinweises auf die Patenterteilung: **07.10.2009**
(21) Anmeldenummer: 04802853.4
(22) Anmeldetag: 01.12.2004
(51) Int. Cl.: A61B 17/70, A61B 17/86

(54) **PEDIKELSCHRAUBE**
PEDICLE SCREW
VIS PEDICULAIRE

(30) Priorität: 10.03.2004 DE 102004011612
(43) Veröffentlichungstag der Anmeldung: 22.11.2006
(73) Patentinhaber: Ulrich GmbH & Co. KG, 89081 Ulm (DE)
(72) Erfinder: SUTCLIFFE, John, Essex CM14PR (GB); MACK, Thomas, 74564 Crailsheim (DE); RICHTER, Marcus, 65191 Wiesbaden (DE); WILLMANN, Nicholas, 89077 Ulm (DE)
(74) Vertreter: Hentrich, Swen
(86) Internationale Anmeldenummer: PCT/DE2004/002644
(87) Internationale Veröffentlichungsnummer: WO 2005/087120

(56) Entgegenhaltungen:
- EP-A- 0 305 417
- EP-A- 1 323 391
- WO-A-01/26568
- WO-A-20/04047657
- WO-A-20/04103194
- US-A1- 2003 004 511

## Beschreibung

Die Erfindung betrifft eine Pedikelschraube für Implantate zur Korrektur und Stabilisierung der Wirbelsäule, mit einem an dem einen axialen Ende eines Gewindeschaftes angeordneten Kopfteil, an dem ein Bügelteil anschließbar ist, dass eine Bügelaufnahme für einen am Kopfteil fixierbaren Stab aufweist.

Derartige Pedikelschrauben sind aus der Praxis bekannt, die dazu dienen, in die Wirbelkörper einer Wirbelsäule eingeschraubt zu werden, um bei degenerativen oder traumatischen Erkrankungen der Wirbelsäule die Ausübung ihrer Funkion zu ermöglichen, indem mindestens ein mehrere Wirbelkörper überbrückender Stab an der Wirbelsäule entlang geführt und an dieser verankert wird. Für die präzise Ausrichtung des Stabes ist es erforderlich, die Lage der Pedikelschraube zumindest an der Kontaktstelle an den Stab anpassen zu können unter Berücksichtigung der anatomisch richtigen Lage der Pedikelschraube innerhalb des Wirbelkörpers. In der Praxis werden daher für eine Operation sehr viele Pedikelschrauben bereit gehalten, durch die die unterschiedliche Größe der Wirbelkörper in Abhängigkeit ihrer Lage in der Halswirbelsäule, der Brustwirbelsäule oder der Lendenwirbelsäule berücksichtigt werden konnte, was die Kosten in der Herstellung, der Lagerhaltung und der Bereitstellung für die Operation erhöht. Die Offenlegungsschrift WO 2004/103194, die unter Art. 54(3) EPÜ fällt, offenbart eine Pedikelschraubenanordnung, die eine einstellbare Sicherung eines Fixierungsstabes zwischen zwei Wirbelkörpern ermöglicht. Die Anordnung umfasst eine Pedikelschraube mit einem kugelförmigen Kopfteil, einem ein Gewinde aufweisenden Schaftbereich und einer Werkzeugeingrifffläche in dem Kopfbereich, um die Schraube in den Wirbelkörper zu treiben.

Die Europäische Patentanmeldung EP-A-1 323 391 offenbart ebenfalls eine Pedikelschraube. Diese weist einen Kopfteil auf, der an dem einen axialen Ende eines Gewindeschaftes angeordnet ist, an dem ein Bügelteil anschließbar ist. Das Bügelteil weist eine Bügelaufnahme für einen am Kopfteil fixierbaren Stab auf.

Der Erfindung liegt daher die Aufgabe zugrunde, eine Pedikelschraube der eingangs genannten Art so auszubilden, dass diese Nachteile vermieden werden können.

Diese Aufgabe wird nach der Erfindung bei einer Pedikelschraube der eingangs genannten Art dadurch gelöst, dass im Bügelteil eine Gewindebohrung für die Aufnahme eines dem Kopfteil zugeordneten Gewindes ausgebildet ist und dass das Kopfteil als Kugelkopf geformt und das Gewinde auf der Außenseite eines Gewindekäfigs ausgebildet ist, dessen Käfiginneres eine Kugelpfanne für den Kugelkopf bildet.

Durch diese Gestaltung ist die Möglichkeit geschaffen, über das Bügelteil eine flexible Verbindung zwischen dem Gewindeschaft und dem Stab bereit zu stellen, so dass eine präzise Positionierung des Stabes möglich ist über die polyaxiale Ausrichtmöglichkeit des Bügelteils mit seiner Bügelaufnahme relativ zu der Achse des Gewindeschaftes.

Ein weiterer Vorteil liegt darin, dass der Gewindeschaft und dessen Bügelteil als separate Bauteile vorliegen, die in unterschiedlichen Varianten bereit gestellt werden können, so dass sich durch die Kombination dieser Varianten eine sehr hohe Anzahl unterschiedlicher Pedikelschraube zusammenstellen lassen. Darüber hinaus ist auch der Vorteil gegeben, dass zunächst ohne Behinderung durch das Bügelteil der Gewindeschaft in den Wirbelkörper eingebracht und dessen richtige Lage kontrolliert werden kann und erst nachfolgend das Bügelteil mit der Gewindebohrung auf den Gewindeschaft aufgeschraubt werden kann.

Um den Gewindeschaft auch unabhängig von dem Bügelteil in den Wirbelkörper einschrauben zu können, ist die Gestaltung so getroffen, dass koaxial zu der Achse des Gewindeschaftes in dem freien Ende des Kopfteils eine nicht-kreisförmige, vorzugsweise vieleckige Werkzeugaufnahme für ein der Ausübung eines Drehmomentes dienendes Werkzeug vorgesehen ist.

Vorteilhaft ist es auch, wenn im Gewindeschaft eine koaxiale Führungsaufnahme ausgebildet ist, von der mindestens ein Seitenkanal zu der Umfangsfläche des Gewindeschaftes führt. Diese Führungsaufnahme bietet die Möglichkeit, einen Bindestoff wie beispielsweise Knochenzement oder andere Klebemittel bei der im Wirbelkörper platzierten Pedikelschraube in die Knochenöffnung einzupressen und so einen besseren, frühzeitig und dauerhaft belastbaren Sitz der Pedikelschraube im Wirbelkörper zu ermöglichen. Zu beachten ist dabei, dass dabei die Beweglichkeit des Bügelteils relativ zu dem Gewindeschaft nicht beeinträchtigt wird, da das Bügelteil noch von dem Gewindeschaft getrennt sein kann und so die Applizierung des Bindestoffes nicht beeinträchtigt und eine entsprechend saubere Arbeitsweise möglich ist, die auch das Entfernen überschüssigen Bindestoffes vor dem Aufschrauben des Bügelteils ermöglicht. Eine schnelle und gleichmäßige Verteilung des Bindestoffes wird erreicht, indem der Seitenkanal mehrfach, gleichmäßig über den Umfang des Gewindeschaftes verteilt oder mehrfach, axial gestaffelt vorgesehen ist.

Bevorzugt ist es weiterhin, wenn der Seitenkanal zwischen den Gewindegängen in die Umfangsfläche des Gewindeschaftes mündet, da so die Gewindeschneiden unbeeinflußt bleiben und das Einschrauben des Gewindeschaftes nicht beeinträchtigt wird.

Wenn die Führungsaufnahme sich über die gesamte axiale Länge des Gewindeschaftes erstreckt, besteht die Möglichkeit, einen vor dem Einschrauben des Gewindeschaftes platzierten Bohrkanal vollständig mit dem Bindestoff auszufüllen. Weiterhin ergibt sich der sehr große Vorteil, dass diese durch die durchgehende Führungsaufnahme kanülierte Pedikelschraube auch in einer "perkutanen" Anwendung im Rahmen minimalinvasiver Chirurgie eingesetzt werden kann, indem zunächst in das Operationsfeld ein Führungsdraht eingebracht wird, auf den der Gewindeschaft mit der Führungsaufnahme aufgefädelt ist, um entlang dieses Führungsdrahtes zu der korrekten Position geführt zu werden.

Zweckmäßiger Weise ist die Anordnung so getroffen, dass die Gewindebohrung im Bügelgrund des Bügelteils angeordnet und durch die Bügelaufnahme ein Ring auf das Kopfteil aufsetzbar ist. Durch diesen Ring wird eine verbesserte Kontaktfläche für den Stab bereit gestellt und darüber hinaus die Möglichkeit geschaffen, durch Aufbringen von externen Druck den Ring auf das Kopfteil zu pressen und so die Beweglichkeit des Kopfteiles gegenüber dem Bügelteil zu beenden.

Um den Stab in der Bügelaufnahme dauerhaft festzulegen, ist an dem ersten Schenkel des Bügelteils über ein Gelenk ein Deckel angeschlossen. Dabei ist es im Rahmen der Erfindung bevorzugt, wenn von dem zweiten Schenkel des Bügelteils radial nach außen, zu der dem ersten Schenkel abgewandten Seite eine Schenkelzunge absteht, in der eine Zungengewindebohrung für die Aufnahme einer den Deckel in einer Öffnung durchsetzenden Sicherungsschraube ausgebildet ist. Bei dieser Gestaltung ergibt sich der Vorteil, dass die Achse der Zungengewindebohrung nicht identisch übereinstimmt mit der Achse des Gewindeschaftes oder der Achse der Gewindebohrung, sondern vielmehr seitlich versetzt ist. Dadurch ist vermieden, dass bei einem Einschrauben der Sicherungsschraube, insbesondere wenn diese ihren festen Sitz erreicht, ein Drehmoment auch auf den Gewindeschaft ausgeübt wird, durch das der Sitz des Gewindeschaftes in den Wirbelkörper wieder gelockert oder der durch die Führungsaufnahme eingepresste Bindestoff beim Abbinden geschädigt wird.

Während einer Operation ergibt sich oft die Notwendigkeit, nach der Festlegung der Lage und Orientierung der Pedikelschraube Feineinstellungen vorzunehmen hinsichtlich der Lage der Wirbelkörper, insbesondere Distraktionen oder Kompressionen auszuführen. Solange dabei aber die Lage des Gewindeschaftes gegenüber dem Kopfteil nicht belastbar festgelegt ist, wirkt eine auf die Wirbelkörper ausgeübte Kraft oftmals nicht distrahierend oder komprimierend, sondern führt zu Kippbewegungen. Um daher die Orientierung des Gewindeschaftes gegenüber dem Bügelteil dauerhaft und belastbar festlegen zu können und dabei eine Drehung des Stabes sowie translatorische Verschiebung gegenüber dem Bügelteil zu ermöglichen, ist zwischen dem Deckel und dem Kopfteil ein Stempel angeordnet zur Klemmung des Kopfteils gegenüber dem Bügelteil beim finalen Schliessen des Deckels. Durch die Klemmung des Kopfteils im Bügelteil ist die relative Lage des Gewindeschaftes zum Bügelteil festgelegt und gesichert, während der Stab gegenüber dem Bügelteil noch verstellt werden kann. Damit ist eine genaue, auch iterative Ausrichtung der Wirbelkörper während der Operation möglich.

Die endgültige Festlegung des Bügelteils gegenüber dem Stab wird in einfacher Weise dadurch erreicht, daß im Deckel eine Schraubenaufnahme ausgebildet ist zur Aufnahme einer der Klemmung des Stabes dienenden Fixierungsschraube.

Zweckmäßigerweise ist der Stempel durch einen am Ring ausgebildeten Ringschenkel gebildet, so daß der zur Klemmung des Kopfteils vorgesehene Ring nicht nur durch den Stab, sondern auch durch den Stempel beaufschlagt wird, der einstückig mit dem Ring ausgebildet ist. Dies reduziert auch die Anzahl der zur Komplettierung der Pedikelschraube erforderlichen Bauteile.

Um eine gleichmäßige Krafteinleitung in den Kopfteil zu erreichen, besteht die Möglichkeit, daß der Ringschenkel zweifach vorgesehen ist auf gegenüberliegenden Seiten des Ringes zum beidseitigen Umfassen des Stabes.

Zur genauen Kraftübertragung von dem Deckel auf den Stempel ist es weiterhin möglich, daß am Bügel auf der dem Kopfteil zugewandten Seite eine Druckleiste ausgebildet zur Beaufschlagung des Stempels.

Um eine große Kraft für die Beaufschlagung des Stempels bereit zu stellen, ist es vorteilhaft, wenn die am Bügelteil ausgebildete Auflagefläche für den Stempel mit Abstand zu dem Gelenk angeordnet ist, also über einen vom Deckel gebildeten Hebelarm eine Kraftverstärkung erzielt werden kann und durch die Länge des Hebelarms ein zusätzlicher Parameter bereit steht für die Bestimmung der Klemmkraft.

Im folgenden wird die Erfindung an in der Zeichnung dargestellte Ausführungsbeispiele näher erläutert; es zeigen:
- Fig. 1: eine Seitenansicht der erfindungsgemäßen Pedikelschraube,
- Fig. 2: den Schnitt II-II aus Fig. 1,
- Fig. 3: in einer Seitenansicht den isolierten Gewindeschaft mit dem als Kugelkopf geformten Kopfteil,
- Fig. 4: eine perspektivische Darstellung des Gewindeschaftes aus Fig. 3,
- Fig. 5: das Detail V aus Fig. 3,
- Fig. 6: eine perspektivische Darstellung des Gewindekäfigs,
- Fig. 7: einen Längsschnitt durch den Gewindekäfig aus Fig. 6,
- Fig. 8: eine perspektivische Darstellung des Rings,
- Fig. 9: einen Längsschnitt durch den Ring aus Fig. 8,
- Fig. 10: eine perspektivische Darstellung des Bügelteils,
- Fig. 11: einen Längsschnitt durch das Bügelteil aus Fig. 10,
- Fig. 12: eine perspektivische Darstellung des Deckels,
- Fig. 13: eine Seitenansicht des Deckels aus Fig. 12,
- Fig. 14: eine perspektivische Darstellung der Sicherungsschraube,
- Fig. 15: eine Explosionsdarstellung der Pedikelschraube,
- Fig. 16: einen Längsschnitt durch den oberen Bereich einer weiteren Ausführungsform,
- Fig. 17: eine Vorderansicht des Bügelteils mit dem Deckel der weiteren Ausführungsform,
- Fig. 18: den Schnitt XVIII-XVIII aus Figur 17,
- Fig. 19: eine perspektivische Darstellung des modifizierten Ringes der weiteren Ausführungsform,
- Fig. 20: den Schnitt XX-XX aus Figur 19,
- Fig. 21: eine perspektivische Darstellung des Deckels der modifizierten Ausführungsform, und
- Fig. 22: den Schnitt XXII-XXII aus Figur 21.

In der Zeichnung ist eine Pedikelschraube 1 für Implantate dargestellt, die aus einem Gewindeschaft 2 und einem Bügelteil 3 besteht. An dem einen axialen Ende des Gewindeschaftes 2 ist ein Kopfteil 4 ausgebildet, das als Kugelkopf geformt ist. Das Bügelteil 3 weist eine Bügelaufnahme 5 für einen am Kopfteil 4 fixierbaren Stab 6 auf, wobei im Bügelgrund 7 des Bügelteils 3 eine Gewindebohrung 8 ausgebildet ist. Weiterhin verfügt das Bügelteil 3 über zwei Schenkel, wobei an dem ersten Schenkel 9 über ein Gelenk 10 ein Deckel 11 angeschlossen ist und von dem zweiten Schenkel 12 radial nach außen, zu der dem ersten Schenkel 9 abgewandten Seite eine Schenkelzunge 13 absteht, in der eine Zungengewindebohrung 14 für die Aufnahme einer den Deckel 11 in einer Öffnung 15 durchsetzenden Sicherungsschraube 16 ausgebildet ist.

Dem Kopfteil 4 ist ein Gewinde zugeordnet, nämlich an der Außenseite eines Gewindekäfigs 26, dessen Käfiginneres eine Kugelpfanne für den Kugelkopf des Kopfteils 4 bildet, wobei das Käfiginnere als Innenkonus oder Teilkugel gestaltet sein kann.

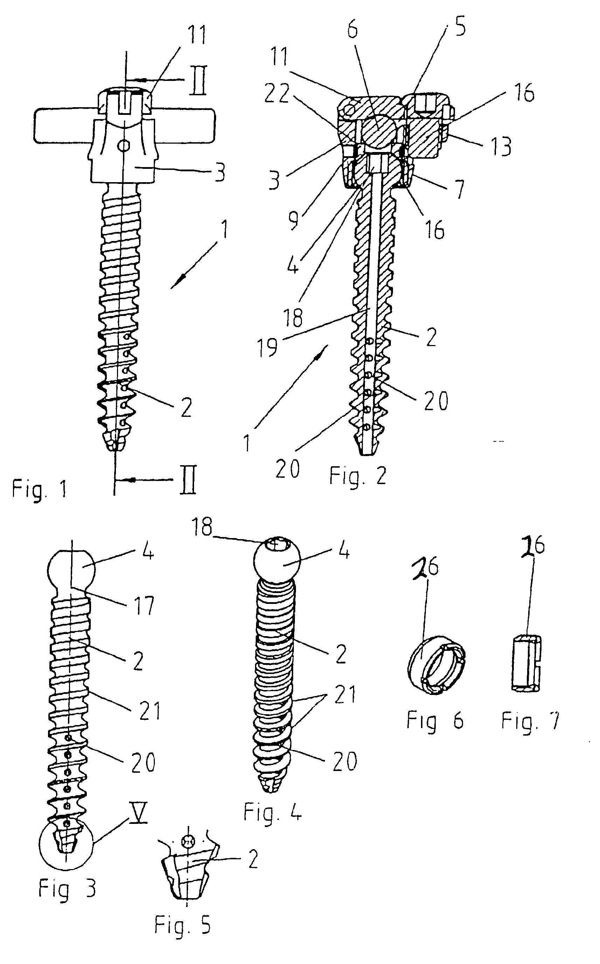

Koaxial zu der Achse 17 des Gewindeschaftes 2 ist in dem freien Ende des Kopfteils 4 eine nicht-kreisförmige Werkzeugaufnahme 18 eingebracht, durch die mittels eines Werkzeuges ein Drehmoment auf den Gewindeschaft 2 ausgeübt und damit der Gewindeschaft 2 in einen Wirbelkörper eingeschraubt werden kann. Der Gewindeschaft 2 verfügt weiterhin über eine koaxiale Führungsaufnahme 19, von der in dem gezeigten Ausführungsbeispiele mehrere, gleichmäßig über den Umfang verteilte, axial gestaffelte Seitenkanäle 20 zu der Umfangsfläche des Gewindeschaftes 2 führen und dort zwischen den Gewindegängen 21 münden. Die Führungsaufnahme 19 erstreckt sich über die gesamte axiale Länge des Gewindeschaftes 2, der an dem Kopfteil 4 entgegengesetzten Ende mit einem selbstschneidenden Gewinde (Fig. 5) versehen ist.

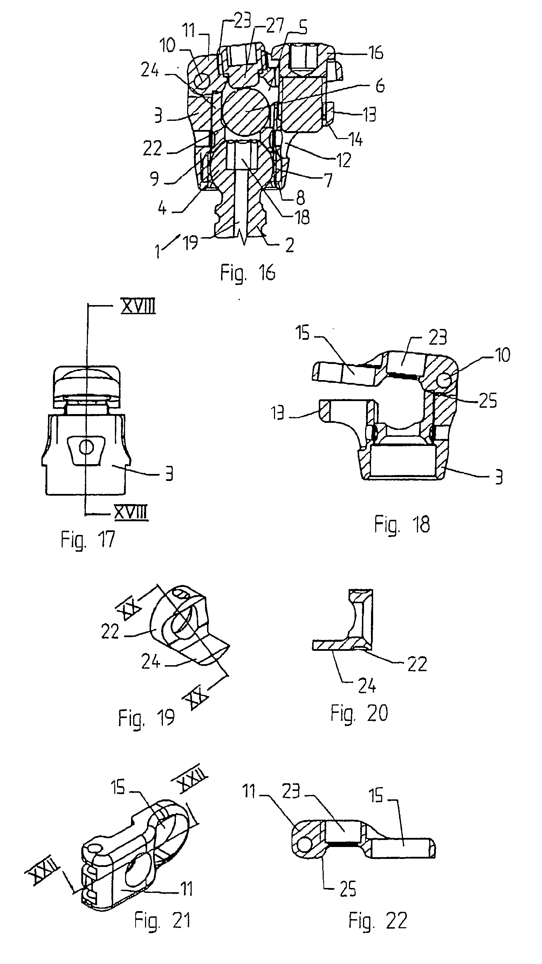

In den Figuren 16 bis 22 ist eine Ausführungsform gezeigt, bei der zwischen dem Deckel 11 und dem Kopfteil 4 ein Stempel 28 angeordnet ist zur Klemmung des Kopfteils 4 gegenüber dem Bügelteil 3 beim finalen Schliessen des Deckels 11, wobei weiterhin im Deckel 11 eine Schraubenaufnahme 23 ausgebildet ist zur Aufnahme einer der Klemmung des Stabes 6 dienenden Fixierungsschraube 27. Dabei ist der Stempel 28 durch einen am Ring 22 ausgebildeten Ringschenkel 24 realisiert, der gemäß einer in der Zeichnung nicht dargestellten Ausführungsform auch zweifach vorgesehen sein kann auf gegenüberliegenden Seiten des Ringes 22 zum beidseitigen Umfassen des Stabes 6.

Insbesondere aus Figur 22 ist ersichtlich, daß am Deckel 11 auf der dem Kopfteil 4 zugewandten Seite eine Druckleiste 25 ausgebildet zur Beaufschlagung des Stempels 28. Die am Deckel 11 ausgebildete Auflagefläche, in Figur 22 die Druckleiste 25, ist für den Stempel 28 mit Abstand zu dem Gelenk 10 angeordnet.

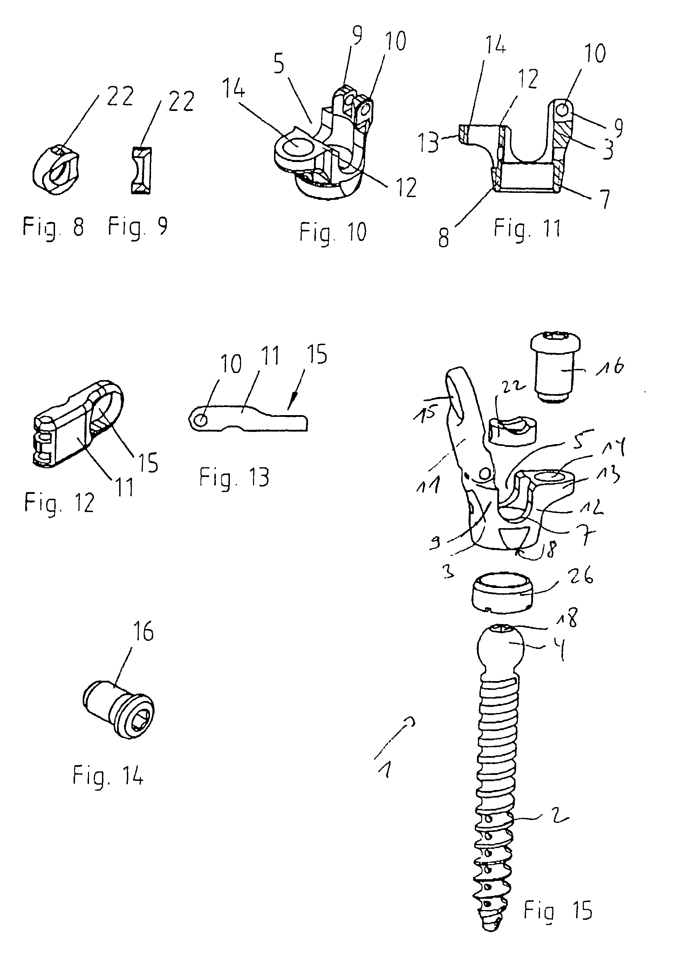

Durch die erfindungsgemäße Gestaltung der Pedikelschraube 1 ergibt sich die Möglichkeit, zunächst den Gewindeschaft 2 in den Wirbelkörper einzuschrauben und dort mittels Knochenzement dauerhaft zu verankern, der durch die Führungsaufnahme 19 und die Seitenkanäle 20 eingepresst wird. Nachfolgend wird dann das Bügelteil 3 an dem Gewindeschaft 2 befestigt, indem das Bügelteil 3 mit der Gewindebohrung 8 auf den Gewindekäfig 16 aufgeschraubt wird. Bei geöffnetem Deckel 11 des Bügelteils 3 wird sodann der Stab 6 auf einen Ring 12 gelegt und beim Verschließen des Deckels 11 durch diesen auf den Ring 22 und damit das Kopfteil 4 des Gewindeschaftes 2 gepresst, umso die Bewegungsmöglichkeit des Bügelteils 3 relativ zu dem Gewindeschaft 2 zu beenden. Mit der Sicherungsschraube 16 erfolgt die Arretierung des Deckels 11 und damit des Stabes 6 bei der ersten Ausführungsform gemäß den Figuren 1 bis 15.

Die Ausführungsform aus den Figuren 16 bis 22 bietet eine gesteigerte Variabilität, da zunächst durch die Sicherungsschraube 16 der Deckel 11 verschlossen werden kann, wodurch auch die Drehbeweglichkeit des Kopfteils 4 gegenüber dem Bügelteil 3 beendet wird. Das Bügelteil 3 selber ist aber noch gegenüber dem Stab 6 verdrehbar, kann also um dessen Längsachse verdreht und auch parallel zu dieser Längsachse verschoben werden. Diese Bewegungsmöglichkeiten werden mittels der Fixierungsschraube 27 eliminiert, wenn die Pedikelschraube 1 die gewünschte Lage relativ zum Wirbelkörper eingenommen hat. Zu beachten ist, daß die Fixierungsschraube 27 unmittelbar auf den Stab 6 einwirkt, der seinerseits über den Ring 22 die Klemmung des Kopfteils 4 verstärkt.

### Bezugszeichenliste

- 1: Pedikelschraube
- 2: Gewindeschaft
- 3: Bügelteil
- 4: Kopfteil
- 5: Bügelaufnahme
- 6: Stab
- 7: Bügelgrund
- 8: Gewindebohrung
- 9: erster Schenkel
- 10: Gelenk
- 11: Deckel
- 12: zweiter Schenkel
- 13: Schenkelzunge
- 14: Zungengewindebohrung
- 15: Öffnung
- 16: Sicherungsschraube
- 17: Achse des Gewindeschafts
- 18: Werkzeugaufnahme
- 19: Führungsaufnahme
- 20: Seitenkanäle
- 21: Gewindegängen
- 22: Ring
- 23: Schraubenaufnahme
- 24: Ringschenkel
- 25: Druckleiste
- 26: Gewindekäfig
- 27: Fixierungsschraube
- 28: Stempel

## Patentansprüche

1. Pedikelschraube (1) für Implantate zur Korrektur und Stabilisierung der Wirbelsäule, mit einem an dem einen axialen Ende eines Gewindeschaftes (2) angeordneten Kopfteil (4), an dem ein Bügelteil (3) anschließbar ist, das eine Bügelaufnahme (5) für einen am Kopfteil (4) fixierbaren Stab (6) aufweist, wobei im Bügelteil (3) eine Gewindebohrung (8) für die Aufnahme eines dem Kopfteil (4) zugeordneten Gewindes ausgebildet ist und das Kopfteil (4) als Kugelkopf geformt und das Gewinde auf der Außenseite eines Gewindekäfigs (26) ausgebildet ist, dessen Käfiginneres eine Kugelpfanne für den Kugelkopf bildet.

2. Pedikelschraube nach Anspruch 1, **dadurch gekennzeichnet, dass** koaxial zu der Achse (17) des Gewindeschaftes (2) in dem freien Ende des Kopfteils (4) eine nicht-kreisförmige, vorzugsweise vieleckige Werkzeugaufnahme (18) für ein der Ausübung eines Drehmomentes dienendes Werkzeug vorgesehen ist.

3. Pedikelschraube nach Anspruch 1 oder 2, **dadurch gekennzeichnet, dass** im Gewindeschaft (2) eine koaxiale Führungsaufnahme (19) ausgebildet ist, von der mindestens ein Seitenkanal (20) zu der Umfangsfläche des Gewindeschaftes (2) führt.

4. Pedikelschraube nach Anspruch 3, **dadurch gekennzeichnet, dass** der Seitenkanal (20) mehrfach, gleichmäßig über den Umfang des Gewindeschaftes (2) verteilt, vorgesehen ist.

5. Pedikelschraube nach Anspruch 3 oder 4, **dadurch gekennzeichnet, dass** der Seitenkanal (20) mehrfach, axial gestaffelt vorgesehen ist.

6. Pedikelschraube nach einem der Ansprüche 3 bis 5, **dadurch gekennzeichnet, dass** der Seitenkanal (20) zwischen den Gewindegängen (21) in die Umfangsfläche des Gewindeschaftes (2) mündet.

7. Pedikelschraube nach einem der Ansprüche 3 bis 6, **dadurch gekennzeichnet, dass** die Führungsaufnahme (19) sich über die gesamte axiale Länge des Gewindeschaftes (2) erstreckt.

8. Pedikelschraube nach einem der Ansprüche 1 bis 7, **dadurch gekennzeichnet, dass** die Gewindebohrung (8) im Bügelgrund (7) des Bügelteils (3) angeordnet und durch die Bügelaufnahme (5) ein Ring (22) auf das Kopfteil (4) aufsetzbar ist.

9. Pedikelschraube nach einem der Ansprüche 1 bis 8, **dadurch gekennzeichnet, dass** an dem ersten Schenkel (9) des Bügelteils (3) über ein Gelenk (10) ein Deckel (11) angeschlossen ist.

10. Pedikelschraube nach Anspruch 9, **dadurch gekennzeichnet, dass** von dem zweiten Schenkel (12) des Bügelteils (3) radial nach aussen, zu der dem ersten Schenkel (9) abgewandten Seite eine Schenkelzunge (13) absteht, in der eine Zungengewindebohrung (14) für die Aufnahme einer den Deckel (11) in einer Öffnung (15) durchsetzenden Sicherungsschraube (16) ausgebildet ist.

11. Pedikelschraube nach Anspruch 9 oder 10, **dadurch gekennzeichnet, dass** zwischen dem Deckel (11) und dem Kopfteil (4) ein Stempel (28) angeordnet ist zur Klemmung des Kopfteils (4) gegenüber dem Bügelteil beim finalen Schließen des Deckels (11).

12. Pedikelschraube nach Anspruch 11, **dadurch gekennzeichnet, dass** im Deckel (11) eine Schraubenaufnahme (23) ausgebildet ist zur Aufnahme einer der Klemmung des Stabes (6) dienenden Fixierungsschraube (27).

13. Pedikelschraube nach Anspruch 11 oder 12, **dadurch gekennzeichnet, dass** der Stempel (28) durch einen am Ring (22) ausgebildeten Ringschenkel (24) gebildet ist.

14. Pedikelschraube nach Anspruch 13, **dadurch gekennzeichnet, dass** der Ringschenkel (24) zweifach vorgesehen ist auf gegenüberliegenden Seiten des Ringes (22) zum beidseitigen Umfassen des Stabes (6).

15. Pedikelschraube nach einem der Ansprüche 11 bis 14, **dadurch gekennzeichnet, dass** am Deckel (11) auf der dem Kopfteil (4) zugewandten Seite eine Druckleiste (25) ausgebildet ist zur Beaufschlagung des Stempels (28).

16. Pedikelschrauben nach einem der Ansprüche 11 bis 15, **dadurch gekennzeichnet, dass** die am Deckel (11) ausgebildete Auflagefläche für den Stempel (28) mit Abstand zu dem Gelenk (10) angeordnet ist.

## Claims

1. A pedicle screw (1) for implants for the correction and stabilisation of the spinal column, comprising a head portion (4) which is arranged at the one axial end of a screwthreaded shaft (2) and to which can be connected a stirrup portion (3) having a stirrup receiving means (5) for a rod (6) fixable to the head portion (4), wherein provided in the stirrup portion (3) is a screwthreaded bore (8) for receiving a screwthread associated with the head portion (4) and the head portion (4) is in the form of a ball head and the screwthread is provided on the outside of a screwthread cage (26), the interior of which forms a ball socket for the ball head.

2. A pedicle screw according to claim 1 **characterised in that** provided coaxially with respect to the axis (17) of the screwthreaded shaft (2) in the free end of the head portion (4) is a non-circular, preferably polygonal tool receiving means (18) for a tool serving to apply a torque.

3. A pedicle screw according to claim 1 or claim 2 **characterised in that** provided in the screwthreaded shaft (2) is a coaxial guide receiving means (19) from which at least one side passage (20) leads to the peripheral surface of the screwthreaded shaft (2).

4. A pedicle screw according to claim 3 **characterised in that** it has a plurality of side passages (20) distributed uniformly over the periphery of the screwthreaded shaft (2).

5. A pedicle screw according to claim 3 or claim 4 **characterised in that** it has a plurality of side passages (20) in axially staggered relationship.

6. A pedicle screw according to one of claims 3 to 5 **characterised in that** the side passage (20) opens into the peripheral surface of the screwthreaded shaft (1) between the thread flights (21).

7. A pedicle screw according to one of claims 3 to 6 **characterised in that** the guide receiving means (19) extends over the entire axial length of the screwthreaded shaft (2).

8. A pedicle screw according to one of claims 1 to 7 **characterised in that** the screwthreaded bore (8) is arranged in the bottom (7) of the stirrup portion (3) and a ring (22) can be fitted on to the head portion (4) through the stirrup receiving means (5).

9. A pedicle screw according to one of claims 1 to 8 **characterised in that** a cover (11) is connected to the first limb (9) of the stirrup portion (3) by way of a hinge (10).

10. A pedicle screw according to claim 9 **characterised in that** projecting radially outwardly from the second limb (12) of the stirrup portion (3) to the side remote from the first limb (9) is a limb tongue portion (13) in which there is a tongue screwthreaded bore (14) for receiving a securing screw (16) passing through the cover (11) in an opening (15).

11. A pedicle screw according to claim 9 or claim 10 **characterised in that** a punch (28) is arranged between the cover (11) and the head portion (4) for clamping the head portion (4) with respect to the stirrup portion upon final closure of the cover (11).

12. A pedicle screw according to claim 11 **characterised in that** provided in the cover (11) is a screw receiving means (23) for receiving a fixing screw (27) serving to clamp the rod (6).

13. A pedicle screw according to claim 11 or claim 12 **characterised in that** the punch (18) is formed by a limb (24) on the ring (22).

14. A pedicle screw according to claim 13 **characterised in that** it has two ring limbs (24) on opposite sides of the ring (22) for embracing the rod (6) on both sides.

15. A pedicle screw according to one of claims 11 to 14 **characterised in that** provided on the cover (11) on the side towards the head portion (4) is a pressure bar (25) for acting on the punch (28).

16. A pedicle screw according to one of claims 11 to 15 **characterised in that** the contact surface provided on the cover (11) for the punch (22) is arranged at a spacing relative to the hinge (10).

## Revendications

1. Vis pédiculaire (1) pour implants de correction et de stabilisation de la colonne vertébrale, comportant, à l'une des extrémités axiales d'une tige filetée (2), une partie tête (4) à laquelle peut être liée une partie étrier (3) pourvue d'un logement d'étrier (5) pour une barre (6) qui peut être bloquée sur la partie tête (4), un trou fileté (8) destiné à recevoir un filetage associé à la partie tête (4) étant aménagé dans la partie étrier (3), la partie tête (4) étant conformée en tête sphérique et le filetage étant aménagé sur la surface extérieure d'une cage filetée (26) dont l'intérieur de cage forme une portée sphérique pour la tête sphérique.

2. Vis pédiculaire selon la revendication 1, **caractérisée en ce qu'**il est prévu, coaxialement à l'axe (17) de la tige filetée (2), dans l'extrémité libre de la partie tête (4), un logement d'outil (18) non circulaire, de préférence polygonal, pour un outil servant à appliquer un couple de rotation.

3. Vis pédiculaire selon la revendication 1 ou 2, **caractérisée en ce que** dans la tige filetée (2) est aménagé un logement de guide (19) coaxial, à partir duquel au moins un canal transversal (20) s'étend en direction de la surface périphérique de la tige filetée (2).

4. Vis pédiculaire selon la revendication 3, **caractérisée en ce qu'**il est prévu plusieurs canaux transversaux (20), uniformément répartis sur le pourtour de la tige filetée (2).

5. Vis pédiculaire selon la revendication 3 ou 4, **caractérisée en ce qu'**il est prévu plusieurs canaux transversaux (20) échelonnés dans la direction axiale.

6. Vis pédiculaire selon une des revendications 3 à 5, **caractérisée en ce que** le canal transversal (20) débouche entre les filets (21) dans la surface périphérique de la tige filetée (2).

7. Vis pédiculaire selon une des revendications 3 à 6, **caractérisée en ce que** le logement de guide (19) s'étend sur la totalité de la longueur axiale de la tige filetée (2).

8. Vis pédiculaire selon une des revendications 1 à 7, **caractérisée en ce que** le trou fileté (8) est disposé dans le fond (7) de la partie étrier (3) et **en ce qu'**une bague (22) peut être placée sur la partie tête (4) au travers du logement d'étrier (5).

9. Vis pédiculaire selon une des revendications 1 à 8, **caractérisée en ce qu'**un couvercle (11) est lié par l'intermédiaire d'une articulation (10) à la première branche (9) de la partie étrier (3).

10. Vis pédiculaire selon la revendication 9, **caractérisée en ce que** sur la deuxième branche (12) de la partie étrier (3), une patte (13) s'étend radialement vers l'extérieur en direction du côté éloigné de la première branche (9), patte dans laquelle est aménagé un trou fileté de patte (14) destiné à recevoir une vis de blocage (16) qui traverse le couvercle (11) par une ouverture (15).

11. Vis pédiculaire selon la revendication 9 ou 10, **caractérisée en ce qu'**entre le couvercle (11) et la partie tête (4) est placé un élément de blocage (28) pour bloquer la partie tête (4) par rapport à la partie étrier lors de la fermeture définitive du couvercle (11).

12. Vis pédiculaire selon la revendication 11, **caractérisée en ce que** dans le couvercle (11) est aménagé un logement de vis (19) destiné à recevoir une vis de blocage (27) servant à bloquer la barre (6).

13. Vis pédiculaire selon la revendication 11 ou 12, **caractérisée en ce que** dans l'élément de blocage (28) est formé par une saillie (24) prévue sur la bague (22).

14. Vis pédiculaire selon la revendication 13, **caractérisée en ce qu'**il est prévu deux saillies (24) sur des côtés en vis-à-vis de la bague (22) aux fins d'enserrer la barre (6) de deux côtés.

15. Vis pédiculaire selon une des revendications 11 à 14, **caractérisée en ce qu'**une nervure de pression (25) est aménagée sur le couvercle (11) sur la face tournée vers la partie tête (4) aux fins de solliciter le tenon (28).

16. Vis pédiculaire selon une des revendications 11 à 15, **caractérisée en ce que** la surface d'appui pour l'élément de blocage (28) aménagée dans le couvercle (11) est située à distance de l'articulation (10).
